(19) **Europäisches Patentamt / European Patent Office / Office européen des brevets**

(11) **EP 4 566 529 A1**

(12) # EUROPEAN PATENT APPLICATION

(43) Date of publication:
**11.06.2025 Bulletin 2025/24**

(21) Application number: **23214824.7**

(22) Date of filing: **07.12.2023**

(51) International Patent Classification (IPC):
**A61B 5/0536** (2021.01) **A61B 5/055** (2006.01)

(52) Cooperative Patent Classification (CPC):
**A61B 5/0536; A61B 5/055**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventor: **KATSCHER, Ulrich Wolfgang**
**Eindhoven (NL)**

(74) Representative: **Philips Intellectual Property & Standards**
**High Tech Campus 52**
**5656 AG Eindhoven (NL)**

(54) **SYSTEMS AND METHODS FOR ELECTRICAL PROPERTIES TOMOGRAPHY**

(57) Methods and systems for electrical properties tomography imaging are presented. Electrical properties such as conductivity and permittivity of intervertebral discs are calculated with improved accuracy and displayed for assessment of clinical personnel.

402    406, 404    408    410    412

Fig. 4

EP 4 566 529 A1

**Description**

**TECHNICAL FIELD**

The subject matter described herein relates to electrical properties tomography, in particular to deriving electrical conductivity and permittivity maps based on measurements with a magnetic resonance imaging system.

**BACKGROUND**

Due to mainly sedentary work, backpain from spinal causes are an increasing medical problem. A thorough diagnosis is based on lumbar puncture, though a replacement of this invasive procedure by non-invasive, quantitative imaging techniques would be highly beneficial.

Quantitative magnetic resonance imaging (MRI) is one of the imaging techniques that potentially could be used for non-invasive diagnosis, in particular Electrical Properties Tomography (EPT).

EPT is an imaging method that uses a magnetic resonance (MR) system to non-invasively derive spatial distribution of conductivity $\sigma$ and permittivity $\varepsilon$ of an imaged subject from measurement of magnitude and phase of the radiofrequency (RF) transmit field B1 (i.e., measurement of complex B1 map), followed by a subsequent postprocessing of the complex B1 map according to Maxwell's equations. An extensive review of EPT is to be found in Leijsen R., et al., "Electrical Properties Tomography: A Methodological Review", Diagnostics 2021, 11, 176, https://doi.org/10.3390/diagnostics11020176, which is hereby incorporated by reference in its entirety.

The information included in this Background section of the specification, including any references cited herein and any description or discussion thereof, is included for technical reference purposes only and is not to be regarded as subject matter by which the scope of the disclosure is to be bound.

**SUMMARY**

In EPT electric conductivity and permittivity is derived by post-processing complex B1 maps measured with standard MR sequences on standard MR systems. Conductivity and permittivity are obtained from calculating numerically the second derivative of the complex B1 map in all three spatial dimensions.

EPT suffers from strong artefacts along tissue boundaries. Due to the thin shape of spinal intervertebral discs (IVDs), standard EPT of IVDs is dominated by these boundary effects, and thus, does not yield reliable results.

The invention proposes to address this shortcoming through deriving electrical properties of spinal IVDs by applying only one of the three spatial derivatives in EPT. The direction of this single spatial derivative varies across the Field of View (FoV) in such a way that it is always parallel to the main axis of each individual spinal intervertebral discs of the plurality of IVDs in a sagittal image.

In an aspect of the invention a method of electrical properties tomography imaging is provided, comprising:

- receiving magnetic resonance imaging data of an anatomy;
- providing a segmentation of the anatomy comprising one or more anatomical regions based on the magnetic resonance imaging data;
- determining an axis of each of the one or more anatomical regions;
- positioning each of the one or more anatomical regions into axial orientation;
- calculating electrical properties tomography characteristics of each of the one or more anatomical regions based on axial orientation;
- outputting the electrical properties tomography characteristics to a display screen.

Determining an axis of each of the one or more anatomical regions is for example determining a longitudinal axis in two-dimensional image, which may be in a sagittal image.

Positioning each of the one or more anatomical regions into axial orientation may be positioning in the orientation of one of the axes of the Cartesian coordinate system, i.e. along the abscissa or the ordinate. For example, the longitudinal axis of each of the one or more segmented anatomical regions is parallel or coinciding with one of the axes of the Cartesian coordinate system. Positioning may comprise rotation. Positioning may further comprise also translation.

Providing segmentation may comprise segmentation of anatomical regions based on an image generated from the magnetic resonance imaging data. Magnetic resonance imaging data may comprise two-dimensional imaging data.

Any of the embodiments of the method may optionally comprise scaling the electrical properties tomography characteristics of each of the one or more anatomical regions, and outputting the electrical properties characteristics may be outputting the scaled electrical properties tomography characteristics.

In any of the embodiments of the method, outputting the electrical properties tomography characteristics may comprise replacing each of the segmented one or more anatomical regions with corresponding electrical properties tomography characteristics.

An embodiment wherein segmentation of anatomical regions is based on magnetic resonance image, the method may further comprise repositioning the electrical properties tomography characteristics of each of the one or more anatomical regions into the original orientation of the respective segmented one or more anatomical regions, and outputting the electrical properties tomography may comprise overlaying the repositioned electrical properties tomography characteristics of the one or more anatomical regions on the magnetic resonance image used for providing the image segmentation.

In an embodiment of the method, image segmentation may comprise dividing the field of view of the anatomy in multiple subsections of fields of view and the one or more anatomical regions are segmented based on selection of at least one of the subsections of fields of view.

In an embodiment of the method, the magnetic resonance imaging data is three-dimensional imaging data. Positioning each of the one or more segmented anatomical regions may comprise individual rotations in two orthogonal planes, according to the orientation of one of the axes of the Cartesian coordinate systems of the two orthogonal plains, e.g., after individual rotation around the left/right axis in a first plane, also rotation around the anterior/posterior axis.

In any of the embodiments, the magnetic resonance imaging data may be received from a magnetic resonance imaging system based on a balanced Fast Field Echo (bFFE) imaging sequence.

In any of the embodiments at least one of the segmented anatomical regions comprises an intervertebral disc. Electrical property such as conductivity and/or permittivity of intervertebral discs are calculated with improved accuracy and displayed for assessment of clinical personnel. Although the examples are disclosed with respect to intervertebral discs, the invention is applicable and contemplated to any anatomical parts or regions of thin shape wherein standard EPT is dominated by boundary effects, e.g., menisci of the knees or other cartilages in anatomical articulations. In some of the examples, sequences with ultrashort echo time, e.g., tending to zero, may be used for providing the magnetic resonance imaging data, which confers further improved reliable phase for cartilage.

In any of the embodiments the electrical properties tomography characteristics comprises at least one of conductivity and permittivity. Iny any of the embodiments the electrical property characteristics may be displayed as conductivity map or permittivity map.

In a further aspect of the invention a system for electrical properties tomography imaging is provided, comprising a computational system configured to perform any of the method embodiments. The system may comprise a memory for storing machine executable instructions, wherein execution of the machine executable instructions causes the computational system to perform any of the method embodiments.

The system may further comprise a magnetic resonance imaging system for providing the magnetic resonance imaging data of the anatomy and a screen display configured to display the electrical properties tomography characteristics.

In yet a further aspect of the invention a computer program is presented, comprising machine executable instructions, wherein execution of the machine executable instructions causes a computational system to perform any of the method embodiments.

Features described above with respect to method embodiments are applicable and are contemplated also for corresponding system embodiments, with similar benefits.

This Summary is provided to introduce a selection of concepts in a simplified form that are further described below in the Detailed Description. This Summary is not intended to identify key features or essential features of the claimed subject matter, nor is it intended to limit the scope of the claimed subject matter. A more extensive presentation of features, details, utilities, and advantages of the EPT system, as defined in the claims, is provided in the following written description of various embodiments of the disclosure and illustrated in the accompanying drawings.

**BRIEF DESCRIPTION OF THE DRAWINGS**

Illustrative embodiments of the present disclosure will be described with reference to the accompanying drawings, of which:

Figure 1 illustrates an example of a medical system;
Figure 2 illustrates a further example of a medical system;
Figure 3 illustrates a flow chart of a method according to the invention;
Figure 4 illustrates an exemplary workflow according to the invention.

**DETAILED DESCRIPTION**

For the purposes of promoting and understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately. Features described in relation to a system, may be implemented in a computer implemented method and/or in a computer program product, in a corresponding manner.

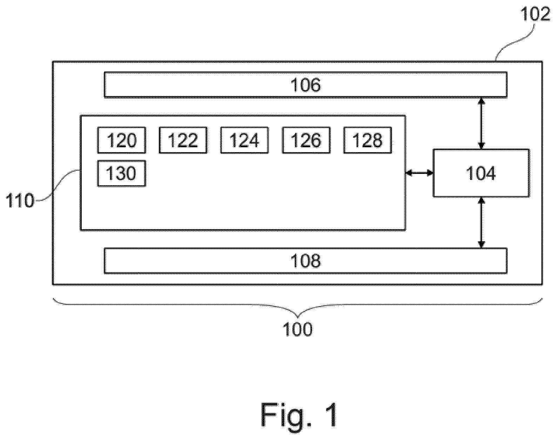

Fig. 1 illustrates an example of a system 100, which may be a medical system. In this example, the system comprises a computer 102. The computer 102 may represent one or more computer systems that are in one location or are distributed. The computer 102 is shown as containing a computational system 104. The computational system 104 may for example be one or more processing cores that are located at one or more locations. The computational system 104 is shown as being connected to an optional hardware interface 106. The hardware interface 106 may for example be used to connect the computational system 104 with other components of the medical system 100 if they are present. The hardware interface 106 may enable the computational system 104 to control such components.

The computer 102 is further shown as comprising an optional user interface 108. The user interface 108 may for example be used by an operator to control the operation and function of the medical system 100. The medical system 100 could be a standalone computer system but it could also be integrated into a magnetic resonance imaging system.

The computer 102 is further shown as comprising a memory 110. The memory 110 is intended to represent any combination of memory or storage device which is accessible to the computational system 104. The memory 110 may include volatile and non-volatile memory storage means and components.

The memory 110 is shown as containing machine-executable instructions 120. The machine-executable instructions 120 may be configured to enable the computational system 104 to perform basic data processing and image processing tasks, such as reconstructing magnetic resonance images. The machine-executable instructions 120 may also in some examples contain code which enables the computational system to control other components via the optional hardware interface 106.

The memory 110 may comprise image segmentation algorithm 122. The image segmentation algorithm 122 may be a standard magnetic resonance imaging image segmentation algorithm that is configured for outputting one or more predetermined anatomical regions for a magnetic resonance imaging data. This may be for a particular field of view or anatomical region of the subject, e.g., one or multiple IVDs of a patient. In one of the examples the segmentation of the IVDs is done based on the magnitude image of the balanced Fast Field Echo (bFFE) scan applied to acquire the B1 phase.

The memory 110 may comprise the measured magnetic resonance imaging data 124. The memory 110 may further comprise an image segmentation 126 that has been received from the image segmentation algorithm 122 by inputting the measured magnetic resonance imaging data 124. The memory 110 may comprise a selected image portion 128. This is one or more regions or anatomical regions that have been identified in the image segmentation 126. The selected image portion 128 may for example be selected using a predetermined criterion that is applied to the image segmentation 126. The memory may comprise one or a plurality of various algorithms 130 for deriving electrical properties based on magnetic resonance imaging data 124 and by optionally using any information present in the memory 110 and/or information that may be input by a user through the user interface 108.

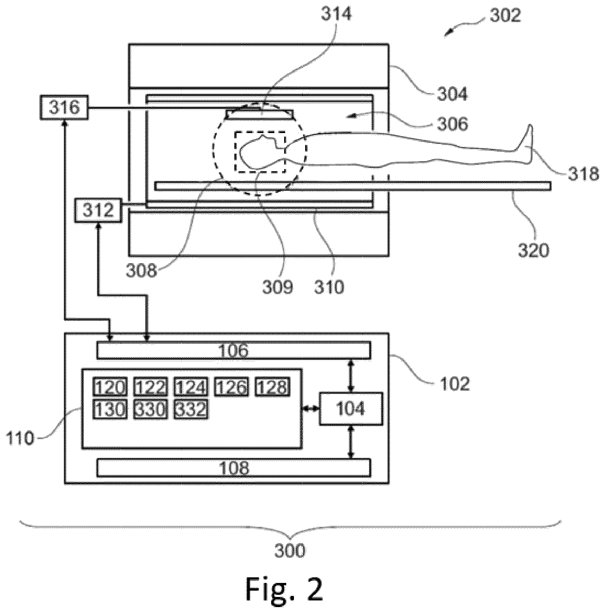

Fig. 2 illustrates a further example of a system 300, which may be a medical system. The medical system 300 comprises the medical system 100 in Fig. 1 and comprises a magnetic resonance imaging (MRI) system 302, or with other words a magnetic resonance imaging scanner, that is controlled by the computational system 104.

The magnetic resonance imaging system 302 comprises a magnet 304. The magnet 304 is a superconducting cylindrical type magnet with a bore 306 through it. The use of different types of magnets is also possible; for instance, it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections is similar to that of a Helmholtz coil. Open magnets are popular, because the subject is less confined. Inside the cryostat of the cylindrical magnet there is a collection of superconducting coils.

Within the bore 306 of the cylindrical magnet 304 there is an imaging zone 308 where the magnetic field is strong and uniform enough to perform magnetic resonance imaging. A region of interest 309 is shown within the imaging zone 308. Magnetic resonance data is typically acquired for the region of interest. A subject 318 is shown as being supported by a subject support 320 such that at least a portion of the subject 318 is within the imaging zone 308 and the region of interest 309.

Within the bore 306 of the magnet there is also a set of magnetic field gradient coils 310 which is used for acquisition of preliminary magnetic resonance data to spatially encode magnetic spins within the imaging zone 308 of the magnet 304. The magnetic field gradient coils 310 are connected to a magnetic field gradient coil power supply 312. The magnetic field gradient coils 310 are intended to be representative. Typically, magnetic field gradient coils 310 contain three separate sets of coils for spatially encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308, there is a radio-frequency coil 314 for manipulating the orientations of magnetic spins within the imaging zone 308 and for receiving radio transmissions from spins also within the imaging zone 308. The radio frequency antenna may contain multiple coil elements. The radio frequency antenna may also be referred to as a channel or antenna. The radio-frequency coil 314 is connected to a radio frequency transceiver 316. The radio-frequency coil 314 and radio frequency transceiver 316 may be replaced by separate transmit and receive coils and a separate transmitter and receiver. It is understood that the radio-frequency coil 314 and the radio frequency transceiver 316 are representative. The radio-frequency coil 314 is intended to also represent a dedicated transmit antenna and a dedicated receive antenna. Likewise, the transceiver 316 may also represent a separate transmitter and receivers. The radio-frequency coil 314 may also have multiple receive/transmit elements and the radio frequency transceiver 316 may have multiple receive/transmit channels.

The transceiver 316 and the gradient controller 312 are shown as being connected to the hardware interface 106 of the computer system 102.

The memory 110 is shown as containing pulse sequence commands 330. The pulse sequence commands are commands or data which can be converted into commands which can be used to control the magnetic resonance imaging system 302 to acquire k-space data 332. An example of pulse sequence comprised by the pulse sequence commands 330 is bFFE. The memory 110 is further shown as comprising k-space data 332 that has been acquired by controlling the magnetic resonance imaging system 302 with the pulse sequence commands 330. The computational system 104 may also reconstruct the measured magnetic resonance imaging data 124 from the k-space data 332.

EPT comprises the determination of one or both electric properties, conductivity and permittivity, and the invention is applicable to both, conductivity and permittivity. For the sake of simplicity, the following description is focused on conductivity $\sigma$ only, which can be reconstructed e.g., from the transceive phase $\varphi$ of a bFFE sequence. The general version of EPT is based on numerically solving the Helmholtz equation

$$\sigma = \nabla^2 \varphi/(2\mu_0\omega) = (\partial_x^2 + \partial_y^2 + \partial_z^2)\varphi/(2\mu_0\omega) \qquad \text{Eq.}\,(1)$$

wherein $\mu_0$ is the magnetic vacuum permeability, $\omega$ is the Larmor frequency.
The partial derivatives in the three spatial directions $x, y, z$ require a volumetric MR image. The specific direction of $x, y, z$ is arbitrary, as long as $x, y, z$ are mutually orthogonal. The approximation

$$\sigma \cong (\partial_x^2 + \partial_y^2)\varphi/(2\mu_0\omega) \qquad \text{Eq.}\,(2)$$

was proposed to allow EPT on fast 2D scans with $x, y$ defining the imaging plane. This invention extends the approximation of Eq. (2) for the investigation of discs by applying

$$\sigma \cong \partial_x^2\varphi/(2\mu_0\omega) \qquad \text{Eq.}\,(3)$$

with $x$ along the main axis parallel to each disc. This approximation avoids the contamination of the reconstructed conductivity with boundary effects from the derivative perpendicular to the disc, which may be significant due to the low number of voxels found in the perpendicular direction.

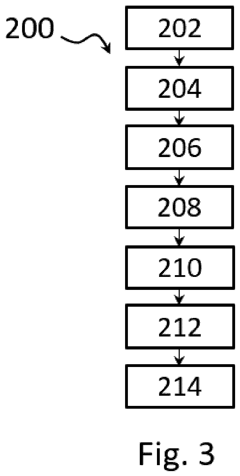

Fig. 3 shows a flowchart, which illustrates a computer-implemented method 200 of operating the medical system 100 illustrated in Fig. 1 or the medical system 300 of Fig. 2. In step 202 magnetic resonance imaging data is received, for example based on a bFFE sequence. Any spin-echo-based sequence may be used for providing the magnetic resonance imaging data, because its transceive phase does not contain the unwanted B0 phase component. Although Ultrashort Echo Time (UTE) and Zero Echo Time (ZTE), developed for imaging tissue with (very) short relaxation times, are based on gradient echo sequences, they are also suitable for providing the magnetic resonance imaging data to carry out the invention, because the B0 phase component is in first order proportional to the echo time, and it is negligible for ultrashort/zero echo time.

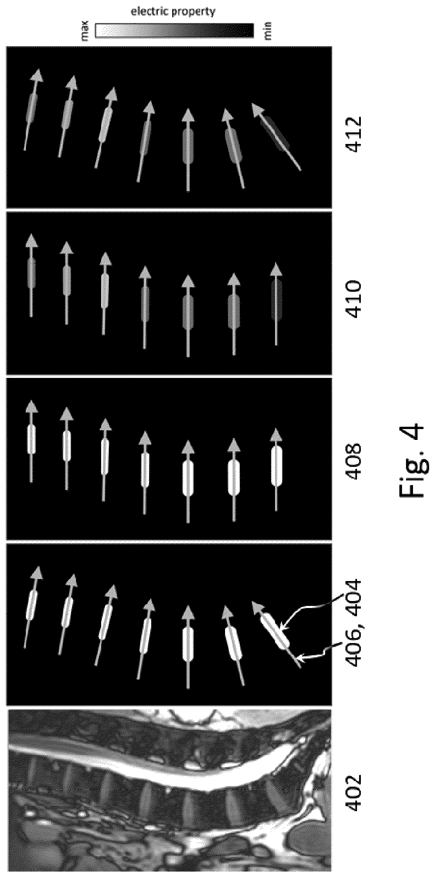

Optionally, an image, e.g., an anatomic image, may be reconstructed or generated based on the magnetic resonance imaging data such as illustrated with 402 in Fig. 4. Segmentation of some or all intervertebral discs of interest is performed in step 204, as illustrated by 404. This can be done, for example, based on the magnitude image of the bFFE scan applied to acquire the B1 phase. Alternatively, image segmentation can be performed with any segmentation technique known in the art that is applicable to magnetic resonance imaging data acquired with any of the hereinbefore mentioned sequences. Subsequently, in step 206 the main axis of each disc is determined, e.g., longitudinal axis in two-dimensional image, as illustrated by 406. Numerical rotation of each disc into a purely axial orientation is performed in step 208, e.g., the main axis of each of the one or more discs pointing in anterior-posterior direction, as illustrated by 408. Typically, each disc has a different rotation angle, with central discs having rotation angles close to zero, and cranial / caudal discs having rotation angles up to $\pm 30°$, wherein cranial and caudal discs are having opposite sign of rotation angles. Different rotation angles can easily be realized by splitting the FoV such that each sub-FoV contains only a single disc. The rotation of the IVDs to axial orientation is followed in step 210 by applying numerically the second derivative required for EPT for each of the one or more discs in anterior-posterior direction only, see Eq. (3). In this way, boundary artefacts appear only in small areas at the two endings of the disc. This is in contrast to the second derivative in feet-head direction, where boundary artefacts would appear along almost the whole circumference of each disc. After calculation of the one-dimensional (1D) second derivative, the result is scaled as usual, see Eq. (3), by corresponding constants, e.g., by division with the term $(2\mu_0\omega)$, to obtain electric properties in absolute values, yielding the electrical property result as illustrated by conduction values in 410 of Fig. 4.

Subsequently, the reconstruction results may be rotated back to the original orientation of each disc in step 212, like for example illustrated by 412 in Fig. 4, and the different sub-FoVs can be combined to the original FoV, resulting in the configuration resembling the original image either before or right after segmentation of the IVDs from the magnetic resonance imaging data. Since application of only one instead of three derivatives according to the invention, the resulting electric properties are in first order 33% of the true value. This can be compensated optionally by multiplying the result with a correction factor q=3 in step 214. In optional embodiment the electrical property results conform 412 may be overlayed on the magnetic resonance image 402, e.g., anatomic image, preferably without indicating the main axis of each of the IVDs. Colorbar may be output corresponding to the scale of values of electrical properties presented in the generated image.

The above-described individual rotation of each disc is also beneficial if three-dimensional (3D) imaging data of the spine is acquired instead of the described two-dimensional (2D) imaging data. After individual rotation around the left/right axis as for the 2D image, and now also around the anterior/posterior axis if a disc shows a corresponding inclination, the disc planes are oriented axially. This orientation allows two of the three derivatives to be computed, see Eq. (2), with $x$ and $y$ defining the plane of the disc under investigation and thus a more accurate estimation of the electric properties is achieved. The correction factor in this case would reduce to $q$=1.5 in the optional step of 214.

The invention can be applied for diagnostics of the spine, particularly investigations involving IVDs. In any of the embodiments specific IVDs can be selected either by the user for segmentation purpose through a user interface or can be predetermined set of IVDs, such as for example lumbar IVDs, which set may be selected from a user interface menu. The knowledge of electric properties of the IVDs can support the investigation of the main constituents of the IVDs, i.e. water, proteoglycans, and collagen. Their depletion is reported in aging and degenerative IVD diseases.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a magnetic hard disk drive, a solid state hard disk, flash memory, a USB thumb drive, Random Access Memory (RAM), Read Only Memory (ROM), an optical disk, a magneto-optical disk, and the register file of the computational system. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a computational system of a general-purpose computer, special-purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen.

All directional references e.g., upper, lower, inner, outer, upward, downward, left, right, lateral, front, back, top, bottom, above, below, vertical, horizontal, clockwise, counterclockwise, proximal, and distal are only used for identification purposes to aid the reader's understanding of the claimed subject matter, and do not create limitations, particularly as to the position, orientation, or use of the reinforced multi-filar conductor bundle. Connection references, e.g., attached, coupled, connected, and joined are to be construed broadly and may include intermediate members between a collection of elements and relative movement between elements unless otherwise indicated. As such, connection references do not necessarily imply that two elements are directly connected and in fixed relation to each other. The term "or" shall be interpreted to mean "and/or" rather than "exclusive or." The word "comprising" does not exclude other elements or steps, and the indefinite article "a" or "an" does not exclude a plurality. Unless otherwise noted in the claims, stated values shall be interpreted as illustrative only and shall not be taken to be limiting.

Although various embodiments of the claimed subject matter have been described above with a certain degree of particularity, or with reference to one or more individual embodiments, those skilled in the art could make numerous alterations to the disclosed embodiments without departing from the spirit or scope of the claimed subject matter.

Still other embodiments are contemplated. It is intended that all matter contained in the above description and shown in the accompanying drawings shall be interpreted as illustrative only of particular embodiments and not limiting. Changes in detail or structure may be made without departing from the basic elements of the subject matter as defined in the following claims.

**Claims**

1.  A method of electrical properties tomography imaging, comprising:

    - receiving (202) magnetic resonance imaging data (124) of an anatomy;
    - providing (204) segmentation (126) of the anatomy comprising one or more anatomical regions based on the magnetic resonance imaging data;
    - determining an axis (206) of each of the one or more anatomical regions;
    - positioning (208) each of the one or more anatomical regions into axial orientation;
    - calculating electrical properties tomography characteristics of each of the one or more anatomical regions based on axial orientation;
    - outputting the electrical properties tomography characteristics to a display screen.

2.  The method of claim 1, further comprising:

    - scaling the electrical properties tomography characteristics of each of the one or more anatomical regions, wherein outputting the electrical properties characteristics comprises outputting the scaled electrical properties tomography characteristics.

3.  The method of claim 1 or 2, wherein outputting the electrical properties tomography characteristics comprises replacing each of the segmented one or more anatomical regions with corresponding electrical properties tomography characteristics.

4.  The method of any of the preceding claims, wherein providing segmentation comprises segmentation of an image generated based on the magnetic resonance imaging data.

5. The method of claim 4, further comprising

- repositioning (212) the electrical properties tomography characteristics of each of the one or more anatomical regions into original orientation of the respective segmented one or more anatomical regions;
wherein outputting the electrical properties tomography comprises overlaying the repositioned electrical properties tomography characteristics of the one or more anatomical regions on an anatomic image, wherein the anatomic image is preferably the magnetic resonance image used for providing the image segmentation.

6. The method of any of the preceding claims, wherein the magnetic resonance imaging data is two-dimensional imaging data.

7. The method of any of the claims 1 to 5, wherein the magnetic resonance imaging data is three-dimensional imaging data.

8. The method of any of the preceding claims, wherein the magnetic resonance imaging data is received based on a bFFE imaging sequence.

9. The method of any of the preceding claims, wherein at least one of the segmented anatomical regions comprises an intervertebral disc.

10. The method of any of the preceding claims, wherein providing image segmentation comprises dividing field of view of the anatomy in subsections of fields of view and wherein the one or more anatomical regions are segmented based on selection of at least one of the subsections of fields of view.

11. The method of any of the preceding claims, wherein the one or more anatomical regions are selectable by a user through a user interface or wherein the one or more anatomical regions are part of a predetermined set selectable by the user through a menu of the user interface.

12. The method of any of the preceding claims, wherein the electrical properties tomography characteristics comprises at least one of conductivity and permittivity.

13. A system (100, 300) for electrical properties tomography imaging, comprising:

- a computational system (104) configured to perform a method according to any of the claims 1 to 12.

14. The system of claim 13, further comprising:

- a magnetic resonance imaging system for providing the magnetic resonance imaging data of the anatomy;
- a screen display configured to display the electrical properties tomography characteristics.

15. A computer program comprising machine executable instructions, wherein execution of the machine executable instructions causes a computational system to perform a method according to any of the claims 1 to 12.

Fig. 1

Fig. 2

200

| 202 |
| :---: |
| 204 |
| 206 |
| 208 |
| 210 |
| 212 |
| 214 |

Fig. 3

electric property

max — min

402    406, 404    408    410    412

Fig. 4

Europäisches
Patentamt

European
Patent Office

Office européen
des brevets

## EUROPEAN SEARCH REPORT

**Application Number**

**EP 23 21 4824**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2019/242961 A1 (MEINEKE JAN JAKOB [DE] ET AL) 8 August 2019 (2019-08-08) * paragraph [0027]; figure 1 * ----- | 1-15 | INV. A61B5/0536 A61B5/055 |
| A | US 2016/242673 A1 (GRYCHTOL BARTLOMIEJ [DE] ET AL) 25 August 2016 (2016-08-25) * paragraph [0115]; figure 9 * ----- | 1-15 | |
| A | US 2022/327703 A1 (JIA FENGGANG [CN] ET AL) 13 October 2022 (2022-10-13) * paragraph [0086] * ----- | 1-15 | |
| A | ULRICH KATSCHER ET AL: "Electric properties tomography: Biochemical, physical and technical background, evaluation and clinical applications", NMR IN BIOMEDICINE., vol. 30, no. 8, 24 May 2017 (2017-05-24), page e3729, XP0055508183, GB ISSN: 0952-3480, DOI: 10.1002/nbm.3729 * the whole document * ----- | 1-15 | |
|  |  |  | **TECHNICAL FIELDS SEARCHED (IPC)** |
|  |  |  | A61B |

The present search report has been drawn up for all claims

| Place of search | Date of completion of the search | Examiner |
|---|---|---|
| The Hague | 29 April 2024 | Worms, Georg |

EPO FORM 1503 03.82 (P04C01)

## ANNEX TO THE EUROPEAN SEARCH REPORT
## ON EUROPEAN PATENT APPLICATION NO.

EP 23 21 4824

This annex lists the patent family members relating to the patent documents cited in the above-mentioned European search report.
The members are as contained in the European Patent Office EDP file on
The European Patent Office is in no way liable for these particulars which are merely given for the purpose of information.

29-04-2024

| Patent document cited in search report | Publication date | Patent family member(s) | Publication date |
|---|---|---|---|
| US 2019242961 A1 | 08-08-2019 | CN 109791186 A | 21-05-2019 |
| | | DE 112017005080 T5 | 11-07-2019 |
| | | US 2019242961 A1 | 08-08-2019 |
| | | WO 2018065233 A1 | 12-04-2018 |
| US 2016242673 A1 | 25-08-2016 | EP 3052018 A1 | 10-08-2016 |
| | | US 2016242673 A1 | 25-08-2016 |
| | | WO 2015048917 A1 | 09-04-2015 |
| US 2022327703 A1 | 13-10-2022 | CN 111063424 A | 24-04-2020 |
| | | EP 4082022 A1 | 02-11-2022 |
| | | US 2022327703 A1 | 13-10-2022 |
| | | WO 2021129842 A1 | 01-07-2021 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LEIJSEN R. et al.** Electrical Properties Tomography: A Methodological Review. *Diagnostics*, 2021, vol. 11, 176, https://doi.org/10.3390/diagnostics11020176